# EUROPEAN PATENT APPLICATION

(11) **EP 4 145 454 A1**
(43) Date of publication of application: **08.03.2023**
(21) Application number: 22189248.2
(22) Date of filing: 08.08.2022
(51) Int. Cl.: G16H 10/40, G16H 40/20, G01N 35/00

(54) **ANALYSIS SYSTEM, MANAGEMENT SERVER, ANALYSIS DEVICE, AND PROGRAM**

(30) Priority: 01.09.2021 JP 2021142567
(71) Applicant: Shimadzu Corporation, Kyoto-shi Kyoto 604-8511 (JP)
(72) Inventor: SASAKI, Akira, KYOTO, 604-8511 (JP); KAWAKAMI, Daisuke, KYOTO, 604-8511 (JP)
(74) Representative: Mewburn Ellis LLP

(57) **Abstract**

An LIS (1) receives, from a laboratory technician, information for evaluation to designate at least one item of a content to be used by an analysis device (3) for deriving an analysis result. The LIS (1) transmits an analysis instruction and a request for the information for evaluation, to the analysis device (3). The analysis device (3) transmits, to the LIS (1), a value (for example, a reagent name used for analysis) of an item designated by the information for evaluation together with the analysis result. In the LIS (1), the laboratory technician evaluates the analysis result with reference to the value of the item designated by the information for evaluation.

## Description

### BACKGROUND OF THE INVENTION

### Field of the Invention

The present disclosure relates to management of an analysis result of a sample by an analysis device.

### Description of the Background Art

Analysis devices have been used in clinical practice or research to analyze samples (for example, blood or urine). As such an analysis device, for example, liquid chromatograph-tandem mass spectrometer (LC-MS/MS) is known.

A system that manages analysis by the analysis device may be referred to as a laboratory information system (LIS). The LIS receives an instruction from a user (for example, a laboratory technician) to the analysis device. The LIS also presents an analysis result outputted from the analysis device, to the user.

In a hospital, in addition to the LIS, a management device called a hospital information system (HIS) may be further used. The HIS is a system for informing an analysis instruction from a doctor to the LIS. Japanese Patent Laying-Open No. 2020-51838 discloses a configuration for managing analysis by an analysis device by using the LIS and the HIS in a hospital. In this configuration, the LIS reports an analysis result outputted from the analysis device, to the HIS.

### SUMMARY OF THE INVENTION

A user evaluates an analysis result presented by the LIS from the viewpoint of whether or not the analysis result can be used as a final analysis result (for example, for reporting to the HIS). In the evaluation, the user determines whether or not the analysis by the analysis device has been adequately performed. In the technique as described in Japanese Patent Laying-Open No. 2020-51838, when it is determined that the analysis has been adequately performed, the user causes the LIS to report the analysis result to the HIS.

When it is determined that the analysis has not been adequately performed, the user examines information (for example, reagent information, a calibration curve, a mass spectrum, and the like) that may affect the analysis. In such a case, the user has been required to go to the analysis device to examine information that may affect the analysis as necessary. That is, in order to evaluate the analysis result, the user has been required to make extra time and effort to go to the analysis device to examine the above information.

The present disclosure has been made in view of such circumstances, and an object is to provide a technique for enabling a user to easily evaluate an analysis result outputted from an analysis device.

A first aspect of the present invention relates to an analysis system including: an analysis device; and a management server that manages analysis in the analysis device, in which the management server includes: an input unit that receives, for each sample, an input of an analysis instruction and information for evaluation of analysis; and a first communication interface that transmits the analysis instruction and a request for the information for evaluation to the analysis device. The information for evaluation designates at least one item of a content to be used by the analysis device for deriving an analysis result, the analysis device includes: an analyzer that derives an analysis result by analyzing a sample in accordance with the analysis instruction; and a second communication interface that transmits, to the management server, the analysis result and a value of an item designated by the information for evaluation in a content used for deriving the analysis result, and the management server further includes an output unit that outputs the analysis result and the value of the item designated by the information for evaluation.

A second aspect of the present invention relates to a management server for managing an analysis device that analyzes a sample. The management server includes: an input unit that receives, for each sample, an input of an analysis instruction and information for evaluation of analysis, in which the information for evaluation designates at least one item of a content to be used by the analysis device for deriving an analysis result. The management server further includes a communication interface that transmits the analysis instruction and a request for the information for evaluation to the analysis device; and an output unit that outputs an analysis result according to the analysis instruction and outputs a value of an item designated by the information for evaluation, in which the analysis result and the value are transmitted from the analysis device.

A third aspect of the present invention relates to an analysis device for analyzing a sample. The analysis device includes: a communication interface that receives, for each sample, an analysis instruction and a request for information for evaluation of analysis from a management server, in which the information for evaluation designates at least one item of a content to be used by the analysis device for deriving an analysis result of a sample. The analysis device further includes: an analyzer that derives an analysis result by analyzing a sample in accordance with the analysis instruction, and the communication interface transmits, to the management server, the analysis result and a value of an item designated by the information for evaluation.

A fourth aspect of the present invention relates to a program that is executed by a computer of a management server, and causes the computer to execute a method for managing an analysis result by an analysis device. The method includes: receiving, for each sample, an input of an analysis instruction and information for evaluation of analysis, in which the information for evaluation designates at least one content to be used by the analysis device for deriving an analysis result. The method further includes: transmitting the analysis instruction and a request for the information for evaluation to an analysis device; and outputting an analysis result according to the analysis instruction and a value of an item designated by the information for evaluation, in which the analysis result and the value are transmitted from the analysis device.

The foregoing and other objects, features, aspects and advantages of the present invention will become more apparent from the following detailed description of the present invention when taken in conjunction with the accompanying drawings.

### BRIEF DESCRIPTION OF THE DRAWINGS

Fig. 1 is a schematic diagram illustrating a configuration of an analysis system 100 according to an embodiment of the present invention.
Fig. 2 is a diagram illustrating a flow when evaluation received in step (11) of Fig. 1 is "inadequate".
Fig. 3 is a diagram illustrating an example of a hardware configuration of an HIS 5.
Fig. 4 is a diagram illustrating an example of a hardware configuration of an LIS 1.
Fig. 5 is a diagram illustrating an example of a configuration of an analysis device 3.
Fig. 6 is a view illustrating an example of an input screen SC1.
Fig. 7 is a view illustrating another example of input screen SC1.
Fig. 8 is a view illustrating an example of a result screen SC2.
Fig. 9 is a flowchart of processing to be executed in each of HIS 5, LIS 1, and analysis device 3.
Fig. 10 is a flowchart of processing to be executed in each of HIS 5, LIS 1, and analysis device 3.

### DESCRIPTION OF THE PREFERRED EMBODIMENTS

Hereinafter, an embodiment of the present invention will be described in detail with reference to the drawings. In the following description, the same or corresponding parts in the drawings are denoted by the same reference numerals, and the description thereof will not be repeated in principle.

### [1. Configuration of analysis system]

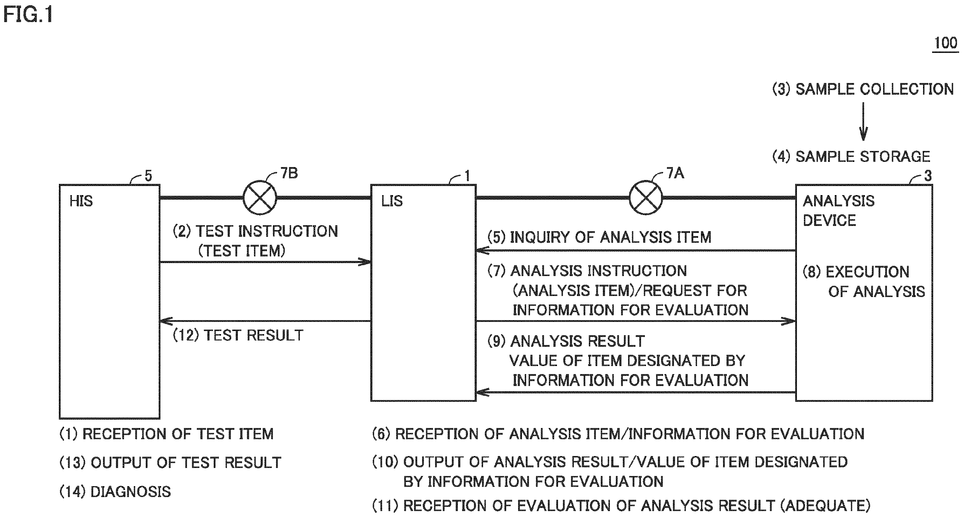

Fig. 1 is a schematic diagram illustrating a configuration of an analysis system 100 according to an embodiment of the present invention. In one implementation example, analysis system 100 is implemented as a system that manages an analysis result of a sample collected from a patient in a medical institution such as a hospital. Analysis system 100 includes an LIS 1, an analysis device 3, and HIS 5.

LIS 1 electronically manages work related to clinical examination. LIS 1 is operated by a laboratory technician, and is exemplified as a server that manages analysis in analysis device 3. The laboratory technician is an example of a user of LIS 1.

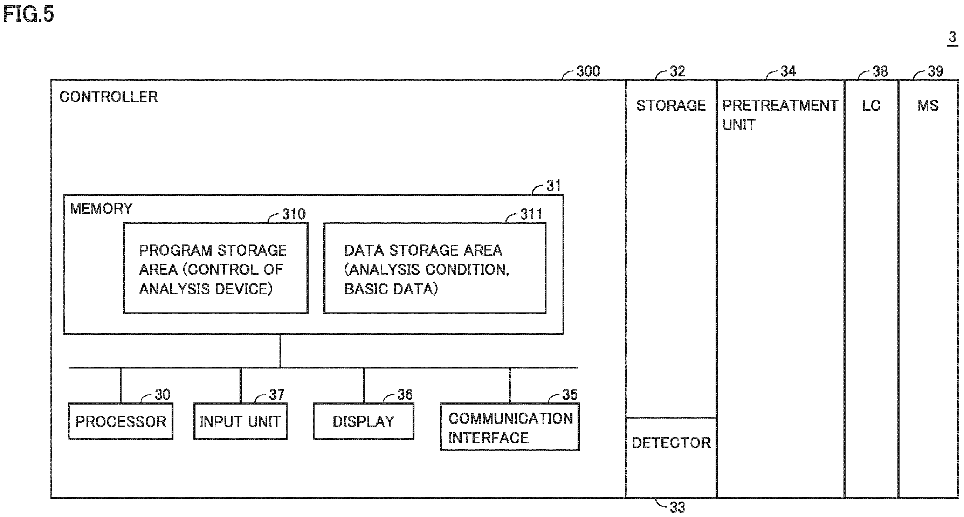

Analysis device 3 analyzes a sample to derive an analysis result of the sample. The sample is, for example, blood, urine, or other metabolites. Analysis device 3 may be LC-MS/MS as described later with reference to Fig. 5, but may be other type of device as long as the device can derive an analysis result of a sample by qualitative analysis and/or quantitative analysis. Analysis device 3 and LIS 1 are connected in a wired or wireless manner. In the example of Fig. 1, analysis device 3 and LIS 1 are connected via a network 7A.

HIS 5 electronically manages work in a hospital, and is also called an electronic medical record. HIS 5 is exemplified as a server that manages test instructions from a doctor or a nurse, test results, and diagnosis contents. HIS 5 and LIS 1 are connected in a wired or wireless manner. In the example of Fig. 1, HIS 5 and LIS 1 are connected via a network 7B.

### [2. Outline of processing in analysis system]

In Fig. 1, (1) to (14) represent an order of processing performed in analysis system 100. Hereinafter, a flow of processing will be described with reference to (1) to (14).

In step (1), HIS 5 receives an input of a test item. The test item specifies a type of value expected to be outputted from analysis device 3. For example, in a case where it is desired to determine whether or not a patient is affected with a certain disease, a doctor requires a blood concentration of a substance to be a marker of the disease. In this case, the doctor inputs the blood concentration of the substance to HIS 5 as the test item.

In step (2), HIS 5 transmits a test instruction to LIS 1. The test instruction includes the test item.

In step (3), a sample is collected from the patient. A type of sample to be collected corresponds to the test item inputted to HIS 5 in step (1). For example, when the test item is a blood concentration of a given substance, blood is collected from the patient. A person who collects the sample from the patient can be specified by the type of sample. For example, when blood is collected as a sample, a person with a given qualification, such as a doctor or a nurse, will collect the sample. When urine is collected as a sample, the sample may be collected by the patient himself/herself.

In step (4), the sample is stored in analysis device 3. The sample is stored, for example, by a laboratory technician.

In step (5), analysis device 3 inquires of LIS 1 about an analysis item of the sample.

In step (6), LIS 1 receives the analysis item and information for evaluation.

Similarly to the test item, the analysis item specifies a type of value expected to be outputted from analysis device 3. The laboratory technician may input the test item transmitted from HIS 5 to LIS 1 in step (2) as it is, to LIS 1 as the analysis item. The laboratory technician may also input the test item included in the test instruction and other items related to the test item, to LIS 1 as the analysis item.

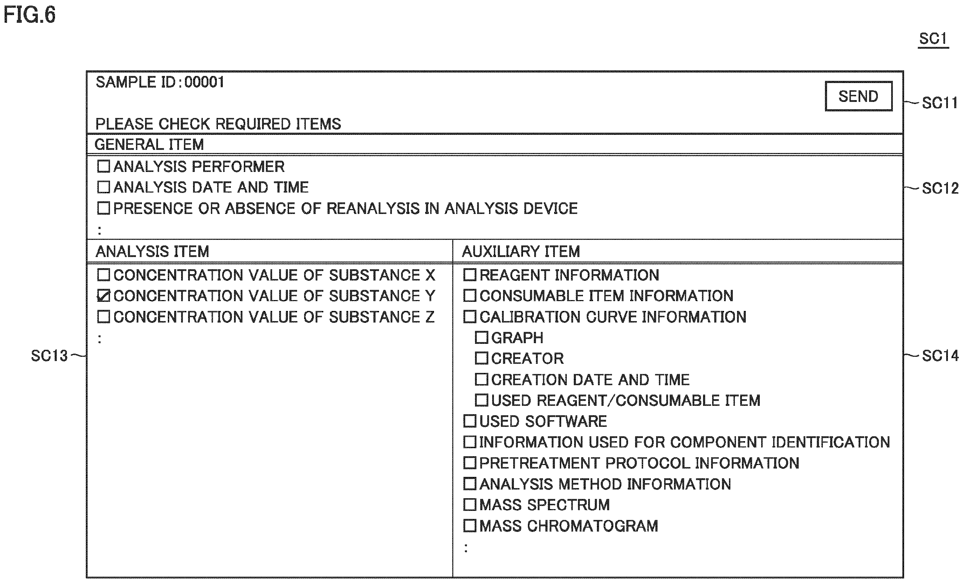

The information for evaluation is information to designate at least one among one or more items to be used for deriving an analysis result in analysis device 3. In one implementation, the information for evaluation designates a reagent to be used for analysis. The one or more items to be used for deriving an analysis result in analysis device 3 and the information for evaluation will be described later with reference to Fig. 6.

In step (7), LIS 1 transmits an analysis instruction and a request for information for evaluation, to analysis device 3. The analysis instruction includes the analysis item inputted in step (6).

In step (8), analysis device 3 analyzes the sample on the basis of the analysis instruction from LIS 1.

In step (9), analysis device 3 transmits the analysis result and a value of an item designated by the information for evaluation, to LIS 1. Analysis device 3 derives an analysis result in accordance with the analysis item included in the analysis instruction from LIS 1, and transmits the analysis result to LIS 1. In analysis device 3, analysis device 3 specifies a value of an item designated by the information for evaluation and transmits the value to LIS 1. For example, when the information for evaluation designates a reagent used for the analysis, analysis device 3 reads information (for example, a reagent name) specifying the reagent from a given storage device in analysis device 3, and transmits the information to LIS 1.

In step (10), LIS 1 outputs the information transmitted from analysis device 3 in step (9). That is, LIS 1 outputs the analysis result and the value of the item designated by the information for evaluation. The laboratory technician confirms the analysis result. The laboratory technician can further evaluate the analysis result by referring to the value of the item designated by the information for evaluation.

In step (11), LIS 1 receives evaluation of the analysis result from the laboratory technician. In one implementation example, a value of either "adequate" or "inadequate" is inputted as the evaluation.

Step (12) is performed in a case where the value "adequate" is inputted as the evaluation. In step (12), LIS 1 transmits, to HIS 5, the analysis result transmitted from analysis device 3 in step (9), as a test result.

In step (13), HIS 5 outputs the test result. The doctor confirms the test result.

In step (14), the doctor makes a diagnosis on the basis of the test result.

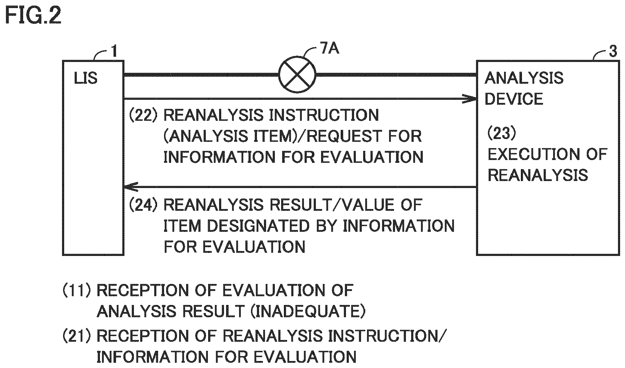

Fig. 2 is a diagram illustrating a flow when evaluation received in step (11) of Fig. 1 is "inadequate". In Fig. 2, (21) to (24) represent an order of processing to be performed after step (11) in analysis system 100. In the example of Fig. 2, after step (11), the processing of LIS 1 proceeds to step (21).

In step (21), LIS 1 receives a reanalysis instruction and information for evaluation from the laboratory technician. The reanalysis instruction includes an analysis item similarly to the analysis instruction in step (7).

In step (22), LIS 1 transmits the reanalysis instruction and a request for information for evaluation, to analysis device 3.

In step (23), analysis device 3 performs reanalysis of the sample on the basis of the reanalysis instruction.

In step (24), analysis device 3 transmits an analysis result of the reanalysis and a value of an item designated by the information for evaluation (step (22)) to LIS 1.

Thereafter, in analysis system 100, the processing proceeds in a flow similar to that in and after step (10) in Fig. 1 described above. That is, LIS 1 outputs the analysis result of the reanalysis and the value of the item designated by the information for evaluation in step (10). The laboratory technician can evaluate the analysis result of the reanalysis by examining the analysis result of the reanalysis and referring to the value of the item designated by the information for evaluation. LIS 1 then receives evaluation of the analysis result from the laboratory technician.

According to the flow described with reference to Figs. 1 and 2, LIS 1 acquires not only the analysis result but also the value of the item designated by the information for evaluation. The value of the item designated by the information for evaluation can be used not only for decision as to whether or not the analysis result can be transmitted to HIS 5 but also for evaluating the analysis result afterwards. This allows the laboratory technician to easily cope with a case where traceability of the analysis result is requested afterwards.

### [3. Hardware configuration]

### (1) HIS

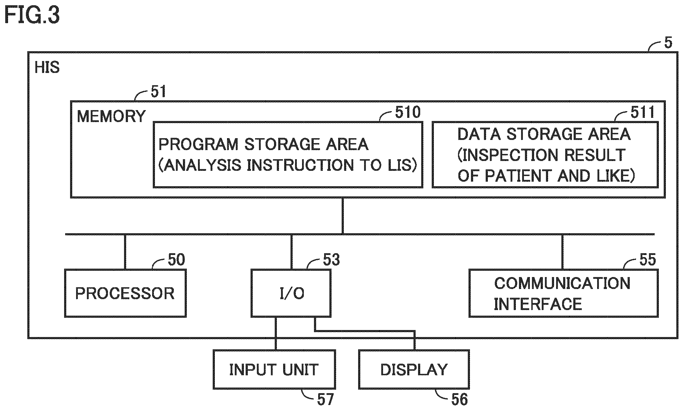

Fig. 3 is a diagram illustrating an example of a hardware configuration of an HIS 5. Referring to Fig. 3, HIS 5 includes a processor 50, a memory 51, an input/output (I/O) interface 53, a communication interface 55, a display 56, and an input unit 57.

Processor 50 controls the entire HIS 5, and is implemented by at least one central processing unit (CPU) in one implementation example.

Memory 51 is implemented by, for example, a read only memory (ROM), a random access memory (RAM), and/or a hard disk drive (HDD). The ROM may store a program to be executed by processor 50. The RAM can temporarily store data to be used during execution of the program in processor 50, and can function as a temporary data memory to be used as a work area. The HDD is a non-volatile storage device. In addition to the HDD or instead of the HDD, a semiconductor storage device such as a flash memory may be adopted. The above-described program and/or data may be stored in an external storage device accessible by processor 50.

Memory 51 includes a program storage area 510 and a data storage area 511. HIS 5 is configured to operate in accordance with a program stored in program storage area 510. Program storage area 510 stores, for example, a program for issuing an analysis instruction to LIS 1. In data storage area 511, for example, an inspection result of a patient is stored.

Communication interface 55 is an interface for HIS 5 to communicate with an external device (for example, LIS 1) in a wireless or wired manner.

I/O interface 53 is an interface for inputting an external signal to HIS 5 or outputting a signal from HIS 5 to an external device. HIS 5 is connected to input unit 57 and display 56 via I/O interface 53.

Input unit 57 receives an input from the laboratory technician. The input includes an instruction to HIS 5. Input unit 57 is implemented by an input device. The input device is, for example, a keyboard, a mouse, and/or a touch panel integrally configured with a display screen of display 56.

Display 56 is an example of a display device, and is, for example, a liquid crystal display.

In the above description, HIS 5 has been described as a single server, but is not limited thereto. HIS 5 may be implemented by cooperation of two or more computers. For example, HIS 5 may be configured by a server including memory 51 and a terminal that communicates with the server. In one implementation, the terminal is owned by the doctor, and includes input unit 57 and display 56.

### (2) LIS

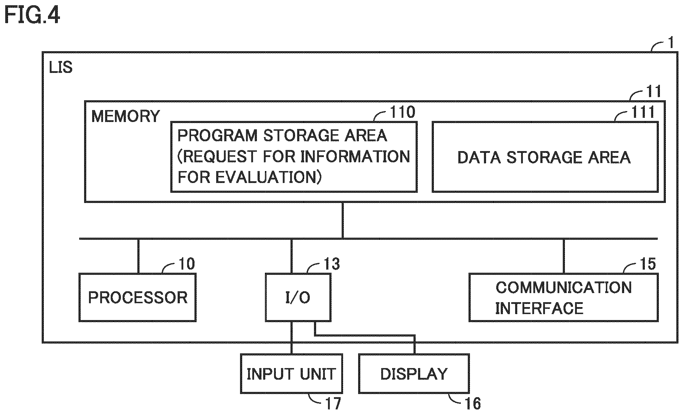

Fig. 4 is a diagram illustrating an example of a hardware configuration of LIS 1. Referring to Fig. 4, LIS 1 includes a processor 10, a memory 11, an I/O interface 13, a communication interface 15, a display 16, and an input unit 17.

Processor 10 controls the entire LIS 1, and is implemented by at least one CPU in one implementation.

Memory 11 is implemented by, for example, a ROM, a RAM, and/or an HDD. The ROM may store a program to be executed by processor 10. The RAM can temporarily store data to be used during execution of the program in processor 10, and can function as a temporary data memory to be used as a work area. The HDD is a non-volatile storage device. In addition to the HDD or instead of the HDD, a semiconductor storage device such as a flash memory may be adopted. The above-described program and/or data may be stored in an external storage device accessible by processor 10.

Memory 11 includes a program storage area 110 and a data storage area 111. LIS 1 is configured to operate in accordance with a program stored in program storage area 110. In program storage area 110, for example, a program related to a request for information for evaluation or the like is stored.

Communication interface 15 is an interface for LIS 1 to communicate with an external device (for example, HIS 5 and analysis device 3).

I/O interface 13 is an interface for inputting an external signal to LIS 1 or outputting a signal from LIS 1 to an external device. LIS 1 is connected to input unit 17 and display 16 via I/O interface 13.

Input unit 17 receives an input from the laboratory technician. The input includes an instruction to LIS 1. Input unit 17 is implemented by an input device. The input device is, for example, a keyboard, a mouse, and/or a touch panel integrally configured with a display screen of display 16.

Display 16 is an example of a display device, and is, for example, a liquid crystal display.

In the above description, LIS 1 has been described as a single server, but is not limited thereto. LIS 1 may be implemented by cooperation of two or more computers. For example, LIS 1 may be configured by a server including memory 11 and a terminal that communicates with the server. In one implementation, the terminal is owned by the laboratory technician, and includes input unit 17 and display 16.

### (3) Analysis device

Fig. 5 is a diagram illustrating an example of a configuration of an analysis device 3. Referring to Fig. 5, analysis device 3 is, for example, LC-MS/MS, and includes a controller 300, a storage 32, a detector 33, a pretreatment unit 34, a liquid chromatograph 38, and a mass analyzer 39. The liquid chromatograph may be written to as LC. The mass analyzer may be written as MS.

Controller 300 controls storage 32, detector 33, pretreatment unit 34, liquid chromatograph 38, and mass analyzer 39. Controller 300 includes a processor 30, a memory 31, a communication interface 35, a display 36, and an input unit 37.

Processor 30 controls the entire analysis device 3, and is implemented by at least one CPU in one implementation.

Memory 31 is implemented by, for example, a ROM, a RAM, and/or an HDD. The ROM may store a program to be executed by processor 30. The RAM can temporarily store data to be used during execution of the program in processor 30, and can function as a temporary data memory to be used as a work area. The HDD is a non-volatile storage device. In addition to the HDD or instead of the HDD, a semiconductor storage device such as a flash memory may be adopted. The above-described program and/or data may be stored in an external storage device accessible by processor 30.

Memory 31 includes a program storage area 310 and a data storage area 311. Analysis device 3 is configured to operate in accordance with a program stored in program storage area 310. Program storage area 310 stores, for example, a program related to control of analysis device 3. Data storage area 311 stores, for example, an analysis condition, basic data, and the like.

Communication interface 35 is an interface for analysis device 3 to communicate with an external device (for example, LIS 1).

Input unit 37 receives an input from the laboratory technician. The input includes an instruction to analysis device 3. Input unit 37 is implemented by an input device. The input device is, for example, a touch panel integrally configured with a display screen of display 36, a button, and/or a switch.

Display 36 is an example of a display device, and is, for example, a liquid crystal display.

Storage 32 stores a sample to be stored in a predetermined container by an operator (for example, a laboratory technician). Analysis device 3 may have a mechanism (a transport system) for automatically storing the sample in the storage 32.

Detector 33 detects the storing of the sample into storage 32, and reads information (a sample ID) for identifying the sample stored in storage 32. Detector 33 includes, for example, an optical sensor for detection of storing of the sample. Detector 33 also includes a barcode reader for detection of the sample ID. For example, an identifier presenting the sample ID is attached to the container of the sample. The identifier may be a barcode or any other type of identifier (a QR code (registered trademark), an IC chip, or the like). The sample ID may be associated with an ID of an analysis target (for example, a patient) from which the sample has been collected, a date and time when the sample has been collected, and/or a type of sample (for example, urine or blood).

Pretreatment unit 34 performs pretreatment for bringing the sample into a state appropriate for measurement by liquid chromatograph 38 and/or mass analyzer 39. The pretreatment includes, for example, at least one of denaturation of a protein in a sample, removal of impurities, extraction or derivatization of a target component, dilution, concentration, enzyme treatment, and centrifugation.

Liquid chromatograph 38 separates the sample for each component.

Mass analyzer 39 may be a tandem mass analyzer, and obtains a mass chromatogram by measurement. Mass analyzer 39 also measures a mass spectrum of each component of the sample introduced from liquid chromatograph 38, and identifies each component of the sample by collating a pattern of the mass spectrum with a given database. Further, by calculating a ratio of a peak value corresponding to each component of the sample to a peak value of a standard substance, mass analyzer 39 outputs a concentration value of each component.

In analysis device 3, liquid chromatograph 38 and/or mass analyzer 39 derives a measurement result of the sample and concentration values of various substances. The concentration value of the substance is an example of the analysis result. In this sense, in analysis device 3, pretreatment unit 34, liquid chromatograph 38, and mass analyzer 39 constitute an analyzer that derives the analysis result. The analyzer performs qualitative analysis and/or quantitative analysis in order to derive the analysis result. The qualitative analysis is, for example, component identification by collating a pattern of a mass spectrum with a database. The quantitative analysis is, for example, measurement of a concentration value of a substance by using a peak area of a mass chromatogram.

In one implementation example, a content used to derive the analysis result in each of pretreatment unit 34, liquid chromatograph 38, and mass analyzer 39 is stored in memory 31. In response to a request for information for evaluation from LIS 1, processor 30 reads a value of an item designated in the information for evaluation, from information stored in memory 31.

In the above description, analysis device 3 has been described as a device in which controller 300, pretreatment unit 34, liquid chromatograph 38, and mass analyzer 39 are integrated, but is not limited thereto. Analysis device 3 may be configured by an assembly of one or more computers and one or more analysis devices. For example, pretreatment unit 34 may be a device independent of liquid chromatograph 38 and mass analyzer 39. Controller 300 may be included in pretreatment unit 34 or may be included in liquid chromatograph 38 and mass analyzer 39. Controller 300 may include a computer having memory 31 and a terminal that communicates with the computer. The terminal includes input unit 37 and display 36.

### [4. Comparison with conventional analysis system]

In a conventional analysis system, a laboratory technician has used an LIS to transmit an analysis instruction for a sample to an analysis device. The analysis may be quantitative analysis (for example, concentration measurement of a specific component) or qualitative analysis (for example, simultaneous screening of multiple components). In response to the instruction described above, the analysis device has transmitted an analysis result to the LIS. The laboratory technician has confirmed the analysis result from the analysis device with the LIS.

When the laboratory technician confirms the analysis result, the laboratory technician may have a doubt about accuracy of the analysis. In such a case, the laboratory technician has been required to go to the analysis device (a place where the analysis device is installed) to confirm a content (for example, a reagent used for analysis, a mass chromatogram, or the like) used for deriving the analysis result. That is, the user has been required to perform additional work of moving to the analysis device for evaluation of the analysis result.

Whereas, in analysis system 100 according to the present embodiment, at least one item of a content used for deriving an analysis result is inputted as information for evaluation. More specifically, the laboratory technician inputs the information for evaluation to LIS 1. In response to this, LIS 1 transmits a request for the information for evaluation to analysis device 3 together with an analysis instruction. Analysis device 3 transmits a value of an item designated by the information for evaluation to LIS 1 together with the analysis result. This allows the laboratory technician to evaluate the analysis result with reference to the value of the item designated by the information for evaluation, together with the analysis result. Since the value of the item designated by the information for evaluation is transmitted to LIS 1 together with the analysis result, the laboratory technician is not required to perform additional work as described above to examine the value. Therefore, analysis system 100 contributes to improvement in work efficiency of quality control of the analysis system.

### [5. User interface]

Next, a user interface displayed in LIS 1 will be described with reference to Figs. 6 to 8. Each screen described in each of Figs. 6 to 8 is displayed on display 16, for example, by processor 10 of LIS 1 executing a given application.

### (1. Input screen)

Fig. 6 is a view illustrating an example of an input screen SC1. Input screen SC1 is displayed to receive an input of an analysis item and information for evaluation from a laboratory technician (step (6) in Fig. 1). Input screen SC1 includes fields SC11 to SC14.

Field SC11 includes a sample ID. Field SC11 further includes a send button for transmitting selection on input screen SC1 by the laboratory technician to analysis device 3.

Field SC12 includes a general item. The general item means an item of general information regarding analysis by analysis device 3. In the example of Fig. 6, items "analysis performer", "analysis date and time", and "presence or absence of reanalysis in analysis device" are shown as the general item.

Field SC13 includes an analysis item. In the example of Fig. 6, concentration values of a plurality of substances (substances X, Y, and Z) are shown as the analysis item.

Field SC14 includes an auxiliary item. The auxiliary item corresponds to the "content to be used for deriving an analysis result" described above.

In the example of Fig. 6, items "reagent information", "consumable item information", "calibration curve information", "used software", "information used for component identification", "pretreatment protocol information", "analysis method information", "mass spectrum", and "mass chromatogram" are shown as the auxiliary item.

The auxiliary item can be classified into an analysis condition and basic data. Each of "reagent information", "consumable item information", "calibration curve information", "used software", "information used for component identification", "pretreatment protocol information", and "analysis method information" is a specific example of the analysis condition. Each of "mass spectrum" and "mass chromatogram" is a specific example of basic data.

The analysis condition means a setting in analysis device 3 for analysis of a sample.

The basic data means data acquired for a sample by analysis device 3 in order to derive a final analysis result of the sample. For example, in order to acquire a concentration value of substance Y in a sample, analysis device 3 obtains a mass chromatogram of the sample. Then, analysis device 3 derives a concentration value of substance Y in the sample by using a peak area value of the mass chromatogram. In this case, the concentration value of substance Y corresponds to the analysis item, and the mass chromatogram corresponds to the basic data.

Each item of the analysis condition will be described. The "reagent information" means a reagent used to derive an analysis result of a sample, and is, for example, a reagent of a standard substance used to derive a concentration value of a given substance in mass analyzer 39.

The "consumable item information" means a consumable item used in analysis device 3, and is, for example, a reagent used for cleaning mass analyzer 39.

The "calibration curve information" means a calibration curve used for deriving an analysis result. In the example of Fig. 6, the "calibration curve information" is subdivided into a graph, a creator, a creation date and time, and used reagent/consumable item. The graph means a graph of a calibration curve. The creator means a creator of the calibration curve. The creation date and time means a date and time when the calibration curve is created. The used reagent/consumable item means a reagent and a consumable item used for creating the calibration curve.

The "used software" means software used to derive an analysis result, or a database including information necessary for component identification. For example, the "used software" is software used in mass analyzer 39 to derive a concentration value for a given substance. An example of this software is software used in mass analyzer 39 to calculate a concentration value of a given substance from a mass chromatogram.

The "Information used for component identification" means information used as a criterion for identification when a component is identified as part of derivation of an analysis result.

The "pretreatment protocol information" means information that defines a protocol to be used in pretreatment unit 34.

The "analysis method information" means information that defines a method used to derive an analysis result.

Each item of basic data will be described. Each of "mass spectrum" and "mass chromatogram" means each of a mass spectrum and a mass chromatogram used to calculate an analysis result (component identification or a concentration value).

In input screen SC1, fields SC13 and SC14 further include check boxes corresponding to the individual items. The laboratory technician designates an item corresponding to the check box as an analysis item, by checking the check box in field SC13. The laboratory technician designates an item corresponding to the check box as information for evaluation, by checking the check box in field SC14.

The items included in input screen SC1 described above with reference to Fig. 6 are merely examples. For example, the items constituting the auxiliary item are not limited to those displayed in field SC14. Items included in field SC12 as the general item may constitute the auxiliary item. In addition, the auxiliary item may include items other than the items included in field SC14. Other items are, for example, a collation library or a specimen type (an unknown specimen/sample for calibration or QC).

Input screen SC1 illustrated in Fig. 6 is displayed at a time point of step (2) in Fig. 1 (a time point when a test instruction is given from HIS 5 to LIS 1). That is, input screen SC1 illustrated in Fig. 6 displays a content of the test instruction (a test item included in the test instruction) transmitted from HIS 5. Therefore, in the state illustrated in Fig. 6, a check box corresponding to an item (a concentration value of substance Y) corresponding to an analysis instruction from a doctor is exclusively checked.

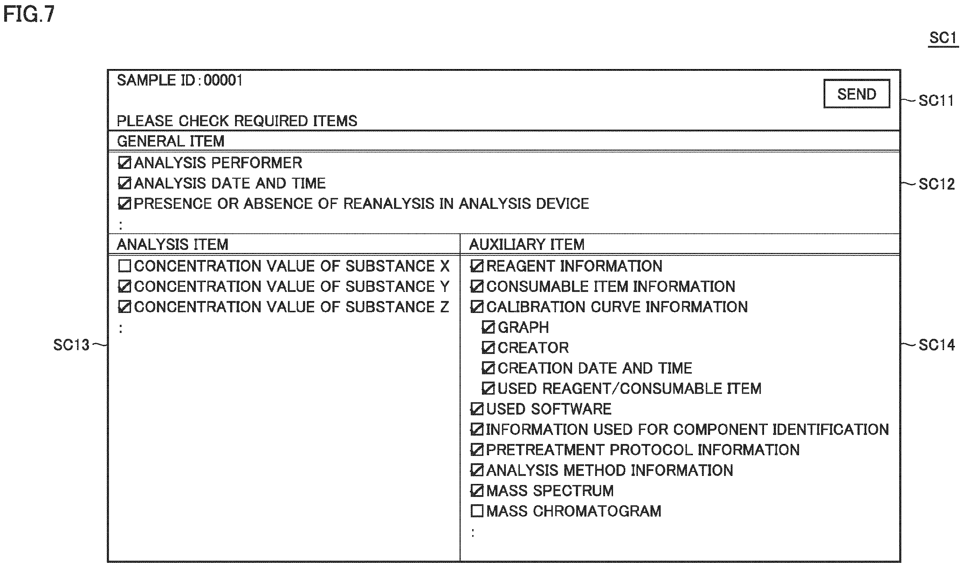

Fig. 7 is a view illustrating another example of input screen SC1. Input screen SC1 illustrated in Fig. 7 is displayed at a time of step (6) in Fig. 1, that is, when LIS 1 receives an analysis item and information for evaluation from the laboratory technician.

In input screen SC1 of Fig. 7, check boxes that have not been checked in the state illustrated in Fig. 6 are also checked. This means that, in step (6) of Fig. 1, the laboratory technician has performed an operation for checking.

For example, in field SC12 of input screen SC1 of Fig. 7, the items "analysis performer", "analysis date and time", and "presence or absence of reanalysis in analysis device", which have not been checked on input screen SC1 of Fig. 6, are also checked. This means that the laboratory technician has checked these items.

Further, in field SC13 of input screen SC1 of Fig. 7, the item "concentration value of substance Z", which has not been checked on input screen SC1 of Fig. 6, is also checked. This means that, when the doctor instructs the "concentration value of substance Y" alone as the test item, the laboratory technician can further designate the "concentration value of substance Z" as the analysis item.

When the send button is operated on input screen SC1 of Fig. 7, LIS 1 transmits a test instruction and a request for information for evaluation to analysis device 3 as described as step (7) of Fig. 1. The test instruction includes the test item. The test item is an item checked in field SC13. The requested information for evaluation designates an item checked in field SC14.

In the example of Fig. 7, LIS 1 receives an input of the information for evaluation by receiving the check box in field SC14. LIS 1 receives an analysis instruction by receiving an operation on the send button. Note that the analysis instruction includes information indicating which check box is checked in field SC13.

### (2. Result screen)

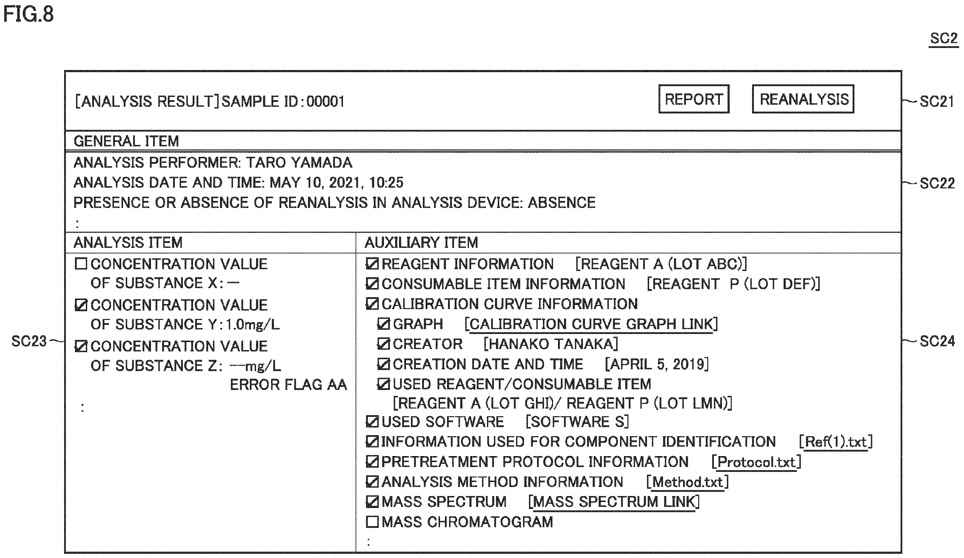

Fig. 8 is a view illustrating an example of a result screen SC2. Result screen SC2 is displayed as an example of an output of the analysis result and the value of the item designated by the information for evaluation described as step (10) in Fig. 1.

As illustrated in Fig. 8, result screen SC2 includes fields SC21 to SC24.

Field SC21 includes a sample ID similarly to field SC11 of input screen SC1. Field SC21 further includes a report button and a reanalysis button. In one implementation example, the report button corresponds to evaluation "adequate" of the analysis result, and the reanalysis button corresponds to evaluation "inadequate" of the analysis result.

When the laboratory technician evaluates that the analysis result is adequate, the laboratory technician operates the report button. Accordingly, LIS 1 transmits the analysis result to HIS 5, as a test result. When the laboratory technician evaluates that the analysis result is inadequate, the laboratory technician operates the reanalysis button. Accordingly, LIS 1 transmits a reanalysis instruction to analysis device 3. The reanalysis instruction includes an analysis item.

In one implementation, the analysis item included in the reanalysis instruction may be the same as the analysis item included in the analysis instruction. In another implementation, LIS 1 may display input screen SC1 again in response to the operation of the reanalysis button. The analysis item set on input screen SC1 at this time may be the analysis item included in the reanalysis instruction.

LIS 1 transmits a request for information for evaluation to analysis device 3 together with the reanalysis instruction. In one implementation example, the information for evaluation targeted by the request transmitted together with the reanalysis instruction may be the same as the information for evaluation targeted by the request transmitted together with the analysis instruction. In another implementation, LIS 1 may display input screen SC1 again in response to the operation of the reanalysis button. The information for evaluation set on input screen SC1 at this time may be the information for evaluation targeted by the request transmitted together with the reanalysis instruction.

Field SC22 includes a general item similarly to field SC12 of input screen SC1, and further includes a value of the general item. For example, field SC22 includes an item "analysis performer" and a value thereof "Taro Yamada". Note that field SC22 includes values exclusively for checked items on input screen SC1 among items included in field SC22.

Field SC23 includes an analysis item similarly to field SC13 of input screen SC1, and further includes a value of the analysis item. The value of the analysis item corresponds to the analysis result. For example, field SC23 includes an item "concentration value of substance Y" and a value "1.0 mg/L". The item "concentration value of substance Y" is an example of the analysis item, and the value "1.0 mg/L" is an example of the analysis result. Field SC23 includes an item "concentration value of substance Z" and a value "error flag AA". The item "concentration value of substance Z" is another example of the analysis item, and the value "error flag AA" is another example of the analysis result. The value "error flag AA" means that analysis of the corresponding analysis item has not been completed normally and a classification (error flag AA) of the cause.

Field SC23 includes values exclusively for checked items on input screen SC1 among items included in field SC23. Field SC23 includes an item "concentration value of substance X" but does not include a value of this item. This means that the check box of the item "concentration value of substance X" has not been checked on input screen SC1.

Field SC24 includes an auxiliary item similarly to field SC14 of input screen SC1, and further includes a value of the auxiliary item. For example, field SC24 includes an item "reagent information" and a value thereof "reagent A (lot ABC)". The "reagent A" is an example of a name of a reagent. The "lot ABC" is information for specifying a lot number used in analysis device 3. That is, analysis device 3 transmits the name of the reagent and the lot number to LIS 1 as the value of the item designated by the information for evaluation, and LIS 1 displays the name of the reagent and the lot number in field SC24.

Field SC24 may indirectly display the value of the item. In the example of Fig. 7, link information (a calibration curve graph link) of a file is shown as a value of an item "graph" in an item "calibration curve information". When the link information is clicked, LIS 1 reads an image file of the calibration curve graph and displays the image file on display 16. In this case, analysis device 3 transmits the image file of the calibration curve graph to LIS 1 as the value of the item designated by the information for evaluation. LIS 1 displays the link information in field SC24 instead of directly displaying the image file.

Field SC24 includes values exclusively for checked items on input screen SC1 among items included in field SC24. Field SC24 includes an item "mass chromatogram" but does not include a value of this item. This means that the check box of the item "mass chromatogram" has not been checked on input screen SC1.

As described with reference to Fig. 7, the auxiliary item includes the item "reagent information". If this item is checked on input screen SC1, the laboratory technician can confirm the name of the reagent on result screen SC2. As a result, the laboratory technician can notice a case where a reagent other than the reagent to be used for analysis of the designated analysis item is erroneously used for the analysis.

In result screen SC2, the value of the item "reagent information" further includes a lot number. As a result, from a reagent lot used for the test, the laboratory technician can notice that an excessively old reagent has been used, or that a reagent of a lot having a concern that an abnormality is found in an analysis result has been used.

The value of the item "graph" on result screen SC2 corresponds to the image file of the calibration curve graph. That is, the laboratory technician can confirm the image file of the calibration curve graph by using result screen SC2. As a result, the laboratory technician can notice a case where the calibration curve graph itself as the basis of the analysis is abnormal.

Result screen SC2 includes values of items "used software", "information used for component identification", "pretreatment protocol information", and" analysis method information". By referring to these values, the laboratory technician can comprehensively consider factors that make the analysis inadequate.

Result screen SC2 further includes a value of basic data ("mass spectrum" and "mass chromatogram"). The value of each of the items "mass spectrum" and "mass chromatogram" is, for example, two-dimensional data. By examining the two-dimensional data as the value of the basic data, the laboratory technician can examine whether or not data measured as a premise of the analysis result is normal in the evaluation of the analysis result. For example, the two-dimensional data is examined in accordance with a viewpoint such as, for example, whether an S/N ratio is normal or whether a waveform of the chromatogram is normal.

In analysis system 100, the laboratory technician can designate an item as information for evaluation for each sample on input screen SC1. Result screen SC2 displays the value of the designated item for each sample. That is, the laboratory technician can obtain the minimum necessary information for each sample. This can avoid, in evaluation of an analysis result, a situation in which the laboratory technician is burdened by excessive information.

As illustrated as the value "error flag AA", the value displayed as the analysis result may indicate a cause of a case where the analysis has not been completed normally. Even in such a case, the laboratory technician can evaluate the analysis result by referring to the value of the auxiliary item displayed in field SC24.

### [6. Flow of processing in analysis system]

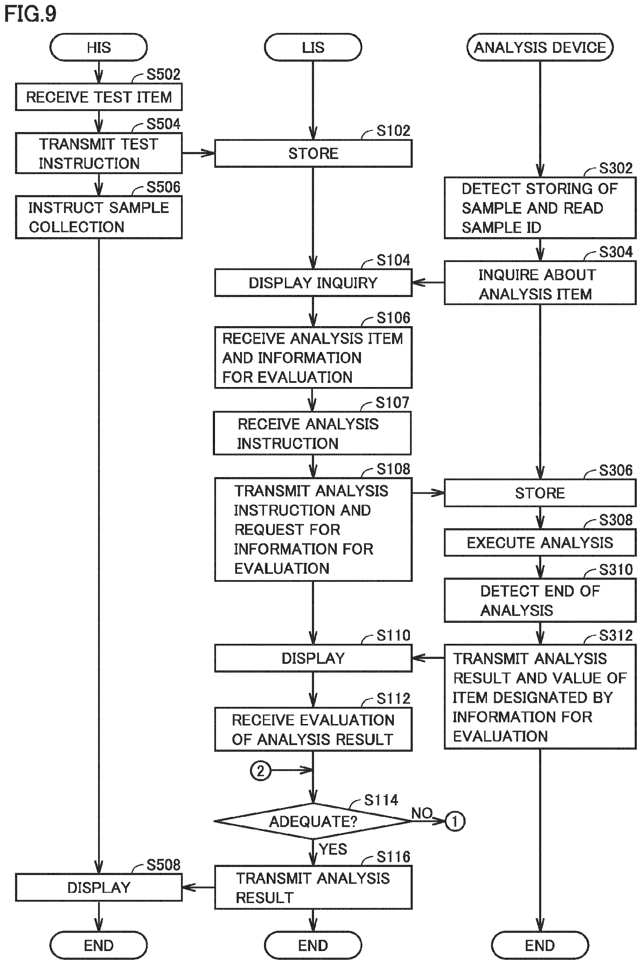

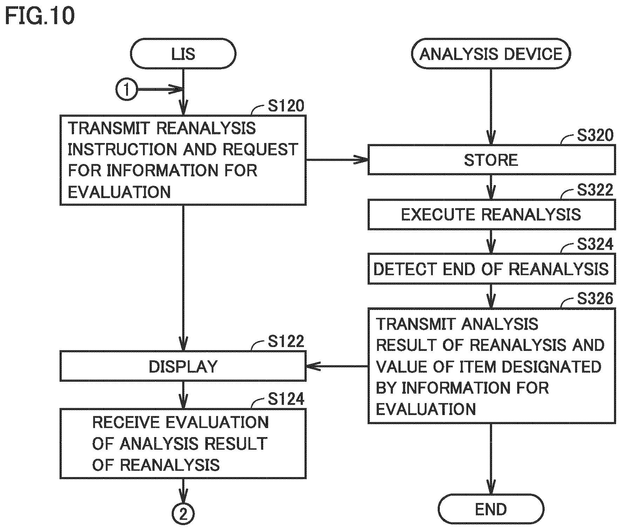

Figs. 9 and 10 are flowcharts of processing to be executed in each of HIS 5, LIS 1, and analysis device 3. In one implementation, the processing illustrated in Figs. 9 and 10 is implemented by executing a given program by each of processor 50 of HIS 5, processor 10 of LIS 1, and processor 30 of controller 300 of analysis device 3.

First, referring to Fig. 9, in step S502, HIS 5 receives an input of a test item from a doctor.

In step S504, HIS 5 transmits a test instruction including the test item to LIS 1.

In step S506, HIS 5 outputs a sample collection instruction. The output of the instruction is, for example, display of the instruction on display 56. On the basis of the instruction from HIS 5, a sample is collected from the patient. In one implementation, a nurse collects the sample from the patient. The collected sample is stored in analysis device 3.

In step S102, LIS 1 stores the test item transmitted from HIS 5, into memory 11.

In step S302, when the storing of the sample is detected, analysis device 3 reads a sample ID from an identifier attached to the sample.

In step S304, analysis device 3 transmits an inquiry about an analysis item corresponding to the read sample ID to LIS 1.

In step S104, LIS 1 displays the inquiry transmitted in step S302 on display 16. In one implementation, the inquiry may be displayed as input screen SC1.

In step S106, LIS 1 receives an analysis item and information for evaluation. In one implementation example, the analysis item and the information for evaluation are received as inputs to input screen SC1.

In step S107, LIS 1 receives an analysis instruction. The analysis instruction is received as, for example, an operation on an operation button of input screen SC1.

In step S108, LIS 1 transmits the analysis instruction and a request for information for evaluation, to analysis device 3.

In step S306, analysis device 3 stores, in memory 31, the analysis item included in the analysis instruction and the request for the information for evaluation transmitted in step S108.

In step S308, analysis device 3 executes analysis based on the analysis item.

In step S310, analysis device 3 detects an end of the analysis in analysis device 3.

In step S312, analysis device 3 transmits an analysis result and a value of an item designated by the information for evaluation, to LIS 1.

In step S110, LIS 1 displays, on display 16, the analysis result and the value of the item designated by the information for evaluation transmitted in step S312. Displaying is an aspect of output.

In step S112, LIS 1 receives the evaluation of the analysis result.

In step S114, LIS 1 determines whether or not evaluation received in step S112 is "adequate". When it is determined as being "adequate" (YES in step S114), LIS 1 advances the control to step S116, otherwise (NO in step S114) LIS 1 advances the control to step S120 (Fig. 10).

In step S116, LIS 1 transmits the analysis result to HIS 5, as a test result.

In step S508, HIS 5 displays the test result transmitted in step S116 on display 56.

Referring to Fig. 10, in step S120, LIS 1 transmits a reanalysis instruction and a request for information for evaluation, to analysis device 3.

In step S320, analysis device 3 stores, in memory 31, the reanalysis instruction and the request for the information for evaluation transmitted in step S120.

In step S322, analysis device 3 executes reanalysis based on the reanalysis instruction.

In step S324, analysis device 3 detects an end of the reanalysis.

In step S326, analysis device 3 transmits, to LIS 1, an analysis result of the reanalysis and a value of an item designated by the information for evaluation.

In step S122, LIS 1 displays, on display 16, the analysis result of the reanalysis and the value of the item designated by the information for evaluation transmitted in step S326.

In step S124, LIS 1 receives evaluation of the analysis result of the reanalysis. Thereafter, LIS 1 returns the control to step S114. When the evaluation of the analysis result of the reanalysis is received, LIS 1 determines whether or not the evaluation of the analysis result of the reanalysis is "adequate" in step S114.

In the processing described with reference to Figs. 9 and 10, LIS 1 displays the value of the item designated by the information for evaluation in addition to the analysis result. By examining the value displayed by LIS 1, the laboratory technician can evaluate the analysis result without moving to analysis device 3. This may reduce a burden on the laboratory technician for establishing and maintaining quality management of analysis in analysis system 100.

### [Aspects]

It is understood by those skilled in the art that the plurality of exemplary embodiments described above are specific examples of the following aspects.

(Clause 1) An analysis system according to one aspect may include an analysis device and a management server that manages analysis in the analysis device. The management server may include: an input unit that receives, for each sample, an input of an analysis instruction and information for evaluation of analysis; and a first communication interface that transmits the analysis instruction and the request for information for evaluation, to the analysis device. The information for evaluation may designate at least one item of a content to be used by the analysis device for deriving an analysis result. The analysis device includes: an analyzer that derives an analysis result by analyzing a sample in accordance with the analysis instruction; and a second communication interface that transmits, to the management server, the analysis result and a value of an item designated by the information for evaluation in a content used to derive the analysis result. The management server may further include an output unit that outputs the analysis result and the value of the item designated by the information for evaluation.

According to the analysis system described in Clause 1, the information for evaluation designates at least one among one or more items of a content to be used by the analysis device for deriving an analysis result. The management server acquires the analysis result and the value of the item designated by the information for evaluation, from the analysis device. A user of the management server can examine the value of the item designated by the information for evaluation, to evaluate the analysis result. As a result, the user can evaluate the analysis result without needing to move to the analysis device.

(Clause 2) In the analysis system according to Clause 1, the at least one item may include at least one of an analysis condition and basic data that is derived for the analysis result by the analyzer.

According to the analysis system described in Clause 2, the user can examine the analysis condition and/or the basic data in the evaluation of the analysis result, as the information for evaluation. The analysis condition and basic data may be directly involved in deriving the analysis result. Therefore, the user can evaluate the analysis result by using information that can be directly involved in deriving the analysis result.

(Clause 3) In the analysis system according to Clause 2, the analysis condition may include at least one of reagent information, consumable item information, calibration curve information, used software, information used for component identification, pretreatment protocol information, and analysis method information.

According to the analysis system described in Clause 3, the user can evaluate the analysis result by using information that can be directly involved in deriving the analysis result.

(Clause 4) In the analysis system according to Clause 2 or 3, the basic data may include at least one of a mass spectrum and a mass chromatogram acquired for a sample.

According to the analysis system described in Clause 4, the user can evaluate the analysis result by using information that can be directly involved in deriving the analysis result.

(Clause 5) The analysis system according to any one of Clauses 1 to 4 may further include an instruction server that transmits a test instruction for a sample, to the management server. The output unit may output a content of the test instruction from the instruction server. An input of the analysis instruction and the information for evaluation may be received after the output unit outputs the test instruction.

According to the analysis system described in Clause 5, a user of the management server can input an analysis instruction and information for evaluation to the input unit on the basis of a content of an instruction from the instruction server.

(Clause 6) In the analysis system according to Clause 5, the input unit may receive evaluation of the analysis result after the output unit outputs the analysis result and the value of the item designated as the information for evaluation, for each sample. The first communication interface may transmit the analysis result to the instruction server for a sample having adequacy as a value of the evaluation.

According to the analysis system described in Clause 6, the analysis result is transmitted from the management server to the instruction server on condition that the analysis result is evaluated as being adequate.

(Clause 7) In the analysis system according to Clause 6, the first communication interface may transmit a reanalysis instruction to the analysis device for a sample having inadequacy as a value of the evaluation.

According to the analysis system according to Clause 7, when a certain analysis result is evaluated as being inadequate, the user can be provided with a new analysis result by reanalysis.

(Clause 8) A management server according to one aspect may be a management server for managing an analysis device that analyzes a sample. The management server may include an input unit that receives, for each sample, an input of an analysis instruction and information for evaluation of analysis. The information for evaluation may designate at least one item of a content to be used by the analysis device for deriving an analysis result. The management server may include: a communication interface that transmits the analysis instruction and a request for the information for evaluation to the analysis device; and an output unit that outputs an analysis result according to the analysis instruction and a value of an item designated by the information for evaluation, in which the analysis result and the value are transmitted from the analysis device.

According to the management server described in Clause 8, the information for evaluation designates at least one among one or more items of a content to be used by the analysis device for deriving an analysis result. The management server acquires the analysis result and the value of the item designated by the information for evaluation, from the analysis device. A user of the management server can examine the value of the item designated by the information for evaluation, to evaluate the analysis result. As a result, the user can evaluate the analysis result without needing to move to the analysis device.

(Clause 9) In the management server according to Clause 8, the at least one item may include at least one of an analysis condition and basic data that is derived for the analysis result by the analysis device.

According to the management server described in Clause 9, the user can examine the analysis condition and/or the basic data in the evaluation of the analysis result, as the information for evaluation. The analysis condition and basic data may be directly involved in deriving the analysis result. Therefore, the user can evaluate the analysis result by using information that can be directly involved in deriving the analysis result.

(Clause 10) In the management server according to Clause 9, the analysis condition may include at least one of reagent information, consumable item information, calibration curve information, used software, information used for component identification, pretreatment protocol information, and analysis method information.

According to the analysis system described in Clause 10, the user can evaluate the analysis result by using information that can be directly involved in deriving the analysis result.

(Clause 11) In the management server according to Clause 9 or 10, the basic data may include at least one of a mass spectrum and a mass chromatogram acquired for a sample.

According to the analysis system described in Clause 11, the user can evaluate the analysis result by using information that can be directly involved in deriving the analysis result.

(Clause 12) In the management server according to any one of Clauses 8 to 11, the communication interface may receive a test instruction for a sample, from an instruction server. The output unit may output a content of the test instruction from the instruction server. An input of the analysis instruction and the information for evaluation may be received after the communication interface receives the test instruction.

According to the management server described in Clause 12, a user of the management server can input an analysis instruction and information for evaluation to the input unit on the basis of a content of the instruction from an instruction server.

(Clause 13) In the management server according to item 12, the input unit may receive, for each sample, evaluation of the analysis result after the output unit outputs a value of an item designated as the information for evaluation. The communication interface may transmit the analysis result to the instruction server for a sample having adequacy as a value of the evaluation.

According to the management server described in Clause 13, the analysis result is transmitted from the management server to the instruction server on condition that the analysis result is evaluated as being adequate.

(Clause 14) In the management server according to Clause 13, the communication interface may transmit a reanalysis instruction to the analysis device for a sample having inadequacy as a value of the evaluation.

According to the management server according to Clause 14, when a certain analysis result is evaluated as being inadequate, the user can be provided with a new analysis result by reanalysis.

(Clause 15) An analysis device according to one aspect may be an analysis device for analyzing a sample. The analysis device may include a communication interface that receives, for each sample, an analysis instruction and a request for information for evaluation of analysis from a management server. The information for evaluation may designate at least one item of a content to be used by the analysis device for deriving an analysis result of a sample. The analysis device may further include an analyzer that derives an analysis result by analyzing a sample in accordance with the analysis instruction. The communication interface may transmit, to the management server, the analysis result and a value of an item designated by the information for evaluation.

According to the analysis device described in Clause 15, the information for evaluation designates at least one among one or more items of a content to be used by the analysis device for deriving an analysis result. The management server acquires the analysis result and the value of the item designated by the information for evaluation, from the analysis device. A user of the management server can examine the value of the item designated by the information for evaluation, to evaluate the analysis result. As a result, the user can evaluate the analysis result without needing to move to the analysis device.

(Clause 16) In the analysis device according to Clause 15, the at least one item may include at least one of an analysis condition and basic data that is derived for the analysis result by the analyzer.

According to the analysis device described in Clause 16, the user can examine the analysis condition and/or the basic data in the evaluation of the analysis result, as the information for evaluation. The analysis condition and basic data may be directly involved in deriving the analysis result. Therefore, the user can evaluate the analysis result by using information that can be directly involved in deriving the analysis result.

(Clause 17) In the analysis device according to Clause 15, the analysis condition may include at least one of reagent information, consumable item information, calibration curve information, used software, information used for component identification, pretreatment protocol information, and analysis method information.

According to the analysis device described in Clause 17, the user can evaluate the analysis result by using information that can be directly involved in deriving the analysis result.

(Clause 18) In the analysis device according to Clause 15, the basic data may include at least one of a mass spectrum and a mass chromatogram acquired for the sample.

According to the analysis device described in Clause 18, the user can evaluate the analysis result by using information that can be directly involved in deriving the analysis result.

(Clause 19) A program according to one aspect may be a program that is executed by a computer of a management server, and causes the computer to execute a method for managing an analysis result by an analysis device. The method may include receiving, for each sample, an input of an analysis instruction and information for evaluation of analysis. The information for evaluation may designate at least one of a content to be used by the analysis device for deriving an analysis result. The method may further include transmitting the analysis instruction and a request for the information for evaluation to the analysis device, and outputting an analysis result according to the analysis instruction and a value of an item designated by the information for evaluation, in which the analysis result and the value are transmitted from the analysis device.

According to the program described in Clause 19, the information for evaluation designates at least one among one or more items of a content to be used by the analysis device for deriving the analysis result. The management server acquires the analysis result and the value of the item designated by the information for evaluation, from the analysis device. A user of the management server can examine the value of the item designated by the information for evaluation, to evaluate the analysis result. As a result, the user can evaluate the analysis result without needing to move to the analysis device.

Although the embodiment of the present invention has been described, it should be considered that the embodiment disclosed herein is illustrative in all respects and not restrictive. The scope of the present invention is defined by the claims, and it is intended to include all modifications within the meaning and scope equivalent to the claims.

## Claims

1. An analysis system (100) comprising:
an analysis device (3); and
a management server (1) that manages analysis in the analysis device (3),
wherein
the management server (1) includes:
an input unit (17) that receives, for each sample, an input of an analysis instruction and information for evaluation of analysis; and
a first communication interface (15) that transmits the analysis instruction and a request for the information for evaluation to the analysis device (3),
the information for evaluation designates at least one item of a content to be used by the analysis device (3) for deriving an analysis result,
the analysis device (3) includes:
an analyzer (34, 38, 39) that derives an analysis result by analyzing a sample in accordance with the analysis instruction; and
a second communication interface (35) that transmits, to the management server (1), the analysis result and a value of an item designated by the information for evaluation in a content used for deriving the analysis result, and
the management server (1) further includes:
an output unit (36) that outputs the analysis result and the value of the item designated by the information for evaluation.

2. The analysis system (100) according to claim 1, wherein the at least one item includes at least one of an analysis condition and basic data that is derived for the analysis result by the analyzer (34, 38, 39).

3. The analysis system (100) according to claim 2, wherein the analysis condition includes at least one of reagent information, consumable item information, calibration curve information, used software, information used for component identification, pretreatment protocol information, and analysis method information.

4. The analysis system (100) according to claim 2 or 3, wherein the basic data includes at least one of a mass spectrum and a mass chromatogram acquired for a sample.

5. The analysis system (100) according to any one of claims 1 to 4, further comprising:
an instruction server (5) that transmits a test instruction for a sample to the management server (1), wherein
the output unit (36) outputs a content of the test instruction from the instruction server (5), and
an input of the analysis instruction and the information for evaluation is received after the output unit (36) outputs the test instruction.

6. The analysis system (100) according to claim 5, wherein
the input unit (17) receives, for each sample, evaluation of the analysis result after the output unit (36) outputs the analysis result and a value of an item designated as the information for evaluation, and
the first communication interface (15) transmits the analysis result to the instruction server (5) for a sample having adequacy as a value of the evaluation.

7. The analysis system (100) according to claim 6, wherein the first communication interface (15) transmits a reanalysis instruction to the analysis device (3) for a sample having inadequacy as a value of the evaluation.

8. A management server (1) for managing an analysis device (3) that analyzes a sample, the management server (1) comprising:
an input unit (17) that receives, for each sample, an input of an analysis instruction and information for evaluation of analysis, the information for evaluation designating at least one item of a content to be used by the analysis device (3) for deriving an analysis result;
a communication interface (15) that transmits the analysis instruction and a request for the information for evaluation to the analysis device (3); and
an output unit (36) that outputs an analysis result according to the analysis instruction and a value of an item designated by the information for evaluation, the analysis result and the value being transmitted from the analysis device (3).

9. The management server (1) according to claim 8, wherein the at least one item includes at least one of an analysis condition and basic data that is derived for the analysis result by the analysis device (3).

10. The management server (1) according to claim 9, wherein the analysis condition includes at least one of reagent information, consumable item information, calibration curve information, used software, information used for component identification, pretreatment protocol information, and analysis method information.

11. The management server (1) according to claim 9 or 10, wherein the basic data includes at least one of a mass spectrum and a mass chromatogram acquired for a sample.

12. The management server (1) according to any one of claims 8 to 11, wherein
the communication interface (15) receives a test instruction for a sample from an instruction server (5),
the output unit (36) outputs a content of the test instruction from the instruction server (5), and
an input of the analysis instruction and the information for evaluation is received after the communication interface (15) receives the test instruction.

13. The management server (1) according to claim 12, wherein
the input unit (17) receives, for each sample, evaluation of the analysis result after the output unit (36) outputs a value of an item designated as the information for evaluation, and
the communication interface (15) transmits the analysis result to the instruction server (5) for a sample having adequacy as a value of the evaluation.

14. The management server (1) according to claim 13, wherein the communication interface (15) transmits a reanalysis instruction to the analysis device (3) for a sample having inadequacy as a value of the evaluation.

15. An analysis device (3) for analyzing a sample, the analysis device (3) comprising:
a communication interface (15) that receives, for each sample, an analysis instruction and a request for information for evaluation of analysis from a management server (1), the information for evaluation designating at least one item of a content to be used by the analysis device (3) for deriving an analysis result of a sample; and
an analyzer (34, 38, 39) that derives an analysis result by analyzing a sample in accordance with the analysis instruction, wherein
the communication interface (15) transmits, to the management server (1), the analysis result and a value of an item designated by the information for evaluation.

16. The analysis device (3) according to claim 15, wherein the at least one item includes at least one of an analysis condition and basic data that is derived for the analysis result by the analyzer (34, 38, 39).

17. The analysis device (3) according to claim 16, wherein the analysis condition includes at least one of reagent information, consumable item information, calibration curve information, used software, information used for component identification, pretreatment protocol information, and analysis method information.

18. The analysis device (3) according to claim 16 or 17, wherein the basic data includes at least one of a mass spectrum and a mass chromatogram acquired for the sample.

19. A program that is executed by a computer of a management server (1), and causes the computer to execute a method for managing an analysis result by an analysis device (3), the method comprising:
receiving, for each sample, an input of an analysis instruction and information for evaluation of analysis, the information for evaluation designating at least one content to be used by the analysis device (3) for deriving an analysis result;
transmitting the analysis instruction and a request for the information for evaluation to the analysis device (3); and
outputting an analysis result according to the analysis instruction and a value of an item designated by the information for evaluation, the analysis result and the value being transmitted from the analysis device (3).
